# EUROPÄISCHE PATENTSCHRIFT

(11) **EP 1 156 746 B1**
(45) Veröffentlichungstag und Bekanntmachung des Hinweises auf die Patenterteilung: **07.08.2002**
(21) Anmeldenummer: 00906375.1
(22) Anmeldetag: 25.02.2000
(51) Int. Cl.: A61B 17/32

(54) **INSTRUMENT ZUM SCHNEIDEN VON BIOLOGISCHEM UND INSBESONDERE MENSCHLICHEM GEWEBE**
INSTRUMENT FOR CUTTING BIOLOGICAL AND NOTABLY HUMAN TISSUE
INSTRUMENT POUR COUPER DES TISSUS BIOLOGIQUES ET NOTAMMENT DES TISSUS HUMAINS

(30) Priorität: 01.03.1999 DE 19908721
(43) Veröffentlichungstag der Anmeldung: 28.11.2001
(73) Patentinhaber: Karl Storz GmbH & Co., 78532 Tuttlingen (DE)
(72) Erfinder: ZISTERER, Uwe, D-78600 Kolbingen (DE); BACHER, Uwe, D-78532 Tuttlingen (DE)
(74) Vertreter: Hofmeister, Frank Horst
(86) Internationale Anmeldenummer: EP0001547
(87) Internationale Veröffentlichungsnummer: WO0051506

(56) Entgegenhaltungen:
- EP-A- 0 555 803
- EP-A- 0 621 008
- WO-A-94/22508
- WO-A-99/07295
- US-A- 5 674 237
- US-A- 5 697 947

## Beschreibung

Die Erfindung betrifft Instrument zum Schneiden von biologischem und insbesondere menschlichem Gewebe, insbesondere bei einem endochirurgischen Eingriff, mit einem Außenrohr und einem in dem Außenrohr angeordneten, um seine Längsachse drehbaren hohlzylindrischen Schneidrohr, an dessen distalem Ende mindestens eine Schneide angeordnet ist, sowie mit einer am proximalen Ende der Rohre angeordneten Handhabe, wobei das Außenrohr in Axialrichtung des unverschiebbar an der Handhabe festgelegten Schneidrohres gegen die Kraft einer Druckfeder zwischen einer Ausgangsstellung, in der das Außenrohr das Schneidrohr distalseitig überragt, und einer Arbeitsstellung, in der die Schneide aus dem distalseitigen Ende des Außenrohres hinausragt, verschiebbar ist.

Derartige, Morcellatoren genannte chirurgische Instrumente werden bei endoskopischen Eingriffen verwendet, die die Entfernung großer Gewebeanteile notwendig machen. Diese Morcellatoren bestehen aus einem angetriebenen Schneidrohr, insbesondere einem elektromechanisch angetriebenen Schneidrohr, das direkt in den Körper bzw. eine natürliche oder künstliche Körperhöhle eingeführt werden kann. Zur Gewebeentfernung wird durch das Schneidrohr eine Faßzange in den Körper bzw. die Körperhöhle eingeführt, mit der das zu extrahierende Gewebe gefaßt wird. Wird die Zange nun zurückgezogen und das Gewebe gegen die Schneidkante des rotierenden Morcellators gepreßt, so kann durch dosierten Zug und gegebenenfalls Variation der Drehgeschwindigkeit und Drehrichtung ein zylinderförmiger Gewebeblock ausgeschnitten und durch das Rohr entfernt werden. Selbst große Gewebemengen können so innerhalb weniger Minuten extrahiert werden.

Ein gattungsgemäßes medizinisches Instrument ist aus EP-A-0 621 008 bekannt. Bei diesem bekannten Instrument kann das Außenrohr durch die Betätigung eines Drückers von der Ausgangsstellung in die Arbeitsstellung überführt werden, in der die Schneide des Schneidrohres das Außenrohr überragt. Durch das Eindrücken des Drückers wird ein Sperrelement verlagert, welches erlaubt, daß das direkt mit dem Drücker verbundene Außenrohr zur Handhabe hin zurückgezogen wird. Da der Drücker und das Außenrohr fest miteinander verbunden sind und der Drücker durch eine Rückstellfeder vorgespannt ist, muß der Operateur den Drücker so lange gedrückt halten, wie die Schneide des Schneidrohres in der Arbeitsstellung verbleiben soll. Aufgrund dieses Umstands ist der Operateur bei der Verwendung dieses bekannten Instruments in seiner Bewegungsfreiheit eingeschränkt, da er den Drücker während des Schneidvorgangs permanent gedrückt halten muß.

Aus der EP-B1-0 555 803 und der EP-A1-0 806 183 sind weitere Morcellatoren bekannt, bei denen innerhalb des Außenrohres ein am distalen Ende über das Außenrohr hinausragendes Schneidrohr angeordnet ist, besteht ebenso wie bei dem aus der DE-C2-44 40 035 bekannten Morcellator, bei dem innerhalb des Schneidrohres ein Schutzrohr angeordnet ist, die Gefahr, daß der Schneidenbereich des Schneidrohres nicht abgedeckt ist bzw. abgedeckt werden kann, so daß Verletzungen durch die Schneide an ungewollten Stellen auftreten können.

Weitere medizinische Schneidinstrumente sind aus der EP-A1-0 841 036 und der EP-A1 0 807 412 bekannt. Aus beiden Druckschriften ist es bekannt, das Außenrohr und das Schneidrohr in Axialrichtung der beiden Rohre relativ zueinander verschiebbar anzuordnen. Nachteilig bei diesen bekannten Instrumenten ist jedoch, daß es bei beiden Instrumenten einer dauerhaften Betätigung des Drückers bedarf, um das Schneidrohr in die Schneidstellung zu überführen. Durch dieses fortwährende Halten des Drückers wird der Operateur deutlich in seiner Bewegungsfreiheit eingeschränkt.

Ausgehend von diesem bekannten Stand der Technik liegt der Erfindung die **Aufgabe** zugrunde, ein chirurgisches Instrument der eingangs genannten Art derart weiterzuentwickeln, daß es bei einfacher und sicherer Handhabbarkeit dem Operateur eine größtmögliche Bewegungsfreiheit gewährleistet.

Die **Lösung** dieser Aufgabenstellung ist erfindungsgemäß dadurch gekennzeichnet, daß das Außenrohr in der Ausgangsstellung über ein Sperrelement gegen die Verschiebung in Axialrichtung gesperrt ist, das am proximalen Ende des Außenrohres anliegt und über einen an der Handhabe angeordneten Betätigungsknopf derart entriegelbar ist, daß das Außenrohr anschließend unabhängig von einer weiteren Betätigung des Betätigungsknopfes durch Ausüben einer axialen Druckkraft auf das Außenrohr in die Arbeitsstellung verschiebbar ist, und daß die Druckfeder das Außenrohr nach jeder Überführung in die Arbeitsstellung automatisch wieder in die gesperrte Ausgangsstellung überführt.

Durch diese erfindungsgemäße Ausgestaltung des chirurgischen Instruments wird dem Operateur eine größtmögliche Bewegungsfreiheit eingeräumt, da er zum Überführen des Außenrohres von der Ausgangsstellung in die Arbeitsstellung nur einmalig den Betätigungsknopf drücken muß, um das am proximalen Ende des Außenrohres anliegende Sperrelement zu entriegeln. Sobald das Sperrelement entriegelt ist, kann der Operateur den Betätigungsknopf wieder loslassen, da das Verschieben des Außenrohres über das mittels eines Faßinstruments durch das hohlzylindrische Schneidrohr ergriffene und zur Handhabe hin gezogene Gewebe oder aber dadurch erfolgt, daß über die Handhabe Druck auf das chirurgische Instrument ausgeübt wird, so daß das als Trokarhülse klemmend von Gewebe umgebene Außenrohr sich gegenüber dem Schneidrohr in die Arbeitsstellung verlagert.

Sobald kein Druck mehr auf das Außenrohr aufgebracht wird, wird das Außenrohr automatisch wieder zurück in die Ausgangsstellung verschoben und in dieser Stellung erneut durch das Sperrelement gesperrt. Ein weiteres Schneiden ist erst wieder möglich, wenn erneut durch Drücken des Betätigungsknopfs das Sperrelement entriegelt wird.

Die Sperrwirkung des Sperrelementes wird weiterhin dadurch verstärkt, daß das Sperrelement in die Sperrstellung federbelastet ist, das heißt, daß das Sperrelement, wenn es nicht über den Betätigungsknopf entriegelt wird, automatisch über eine Druckfeder zurück in die Sperrstellung verlagert wird.

Gemäß einer weiteren Ausführungsform der Erfindung wird das Verriegeln des Außenrohres durch das Sperrelement dadurch erleichtert, daß am proximalen Ende des Außenrohres eine Anlaufschräge für das Sperrelement ausgebildet ist.

Das Entriegeln des Sperrelementes erfolgt bei einer bevorzugten Ausführungsform eines erfindungsgemäßen chirurgischen Instruments über einen fest mit dem Sperrelement verbundenen Stift, der in einer im Schaft des Betätigungsknopfes ausgebildeten Kurvenbahn gelagert ist und beim Drücken des Betätigungsknopfes entlang dieser Kurvenbahn verfahrbar ist. Durch diese Zwangsführung des mit dem Sperrelement kraftschlüssig verbundenen Stiftes wird das Sperrelement bei der Betätigung des Betätigungsknopfes, das heißt beim Eindrücken in das Gehäuse der Handhabe, in die Entriegelungsstellung verlagert.

Schließlich wird mit der Erfindung vorgeschlagen, daß der Betätigungsknopf selber ist gegen die Kraft einer Druckfeder in die Entriegelungsstellung ins Gehäuse der Handhabe eindrückbar, so daß der Betätigungsknopf nach jeder Betätigung automatisch wieder in seine Ausgangsstellung zurückgeführt wird.

Weitere Merkmale und Vorteile der Erfindung ergeben sich aus der nachfolgenden Beschreibung der zugehörigen Zeichnung, in der ein Ausführungsbeispiel eines erfindungsgemäßen Instruments zum Schneiden von biologischem und insbesondere menschlichem Gewebe dargestellt ist. In der Zeichnung zeigt:
- Fig. 1a: eine perspektivische Ansicht eines erfindungsgemäßen Instruments in der Ausgangsstellung;
- Fig. 1b: eine perspektivische Ansicht gemäß Fig. 1a, jedoch in der Arbeitsstellung des erfindungsgemäßen Instruments und
- Fig. 2: einen ausschnittweisen schematischen Längsschnitt durch ein erfindungsgemäßes Instrument in der in Fig. 1a dargestellten Ausgangsstellung.

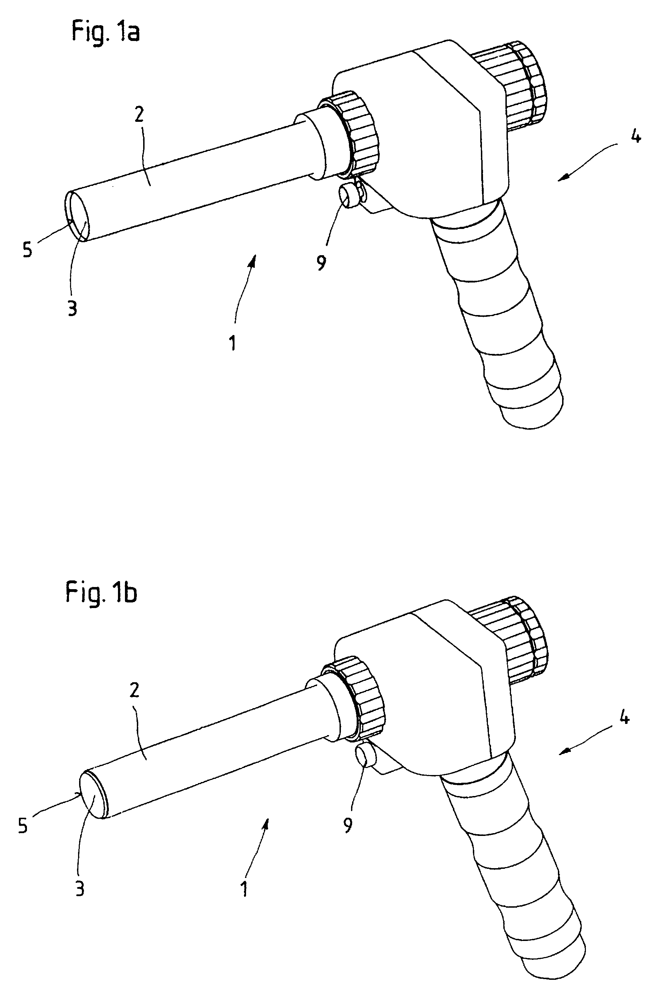

Wie aus Fig. 1a und 1b ersichtlich, besteht das als Morcellator 1 ausgebildete chirurgische Instrument zum Schneiden von biologischem und insbesondere menschlichem Gewebe im wesentlichen aus einem Außenrohr 2 und einem in dem Außenrohr 2 gelagerten Schneidrohr 3, an deren proximalen Enden eine gemeinsame Handhabe 4 angeordnet ist. Das distalseitige Ende des Schneidrohres 3 ist als Schneide 5 zum Schneiden von biologischem und insbesondere menschlichem Gewebe ausgebildet.

Die Abbildungen gemäß Fig. 1a und 1b unterscheiden sich dadurch voneinander, daß in der in Fig. 1a dargestellten Ausgangsstellung des Morcellators 1 das Außenrohr 2 das Schneidrohr 3 distalseitig so überragt, daß die Schneide 5 des Schneidrohres 3 ganz von dem das Schneidrohr 3 koaxial umgebenden Außenrohr 2 abgedeckt wird.

Im Gegensatz hierzu ragt in der in Fig. 1b dargestellten Arbeitsstellung des Morcellators 1 die Schneide 5 des Schneidrohres 3 aus dem distalseitigen Ende des Außenrohres 2 hinaus, so daß die Schneide 5 zum Schneiden von Gewebe verwendet werden kann.

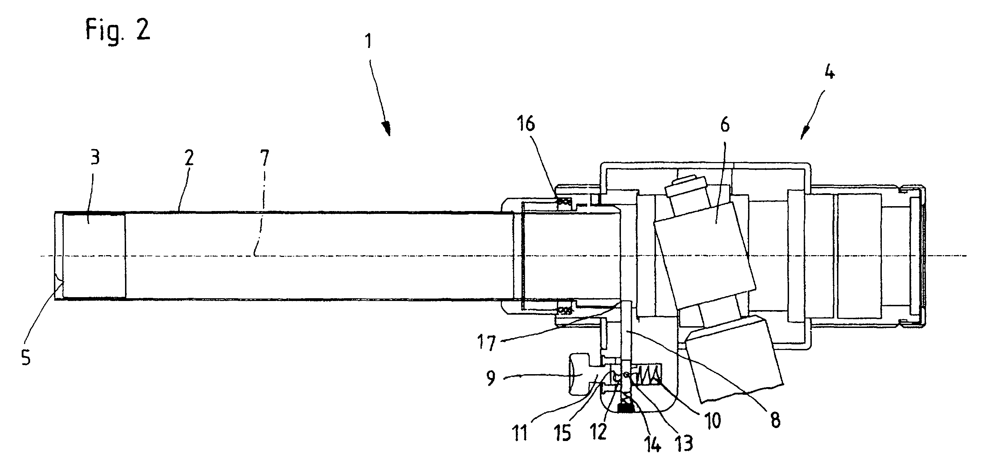

Der genauere Aufbau eines Morcellators ist der Abbildung gemäß Fig. 2 zu entnehmen. Dieser Längsschnitt durch den Morcellator 1 zeigt diesen in der in Fig. 1a dargestellten Ausgangsstellung, in der das Schneidrohr 3 mitsamt der Schneide 5 distalseitig vollständig von dem Außenrohr 2 überragt wird.

Zum Schneiden des biologischen und insbesondere menschlichen Gewebes ist das Schneidrohr 3 über einen Antrieb 6, beispielsweise eine Antriebsschnecke, um seine Längsachse 7 drehbar.

Um den Morcellator 1 von der in Fig. 1a dargestellten Ausgangsstellung in die in Fig. 1b dargestellte Arbeitsstellung zu überführen, ist bei dem dargestellten Ausführungsbeispiel das Schneidrohr 3 in Längsrichtung unverschiebbar mit der Handhabe 4 verbunden, während das Außenrohr 2 relativ zu dem Schneidrohr 3 axial zur Längsachse 7 verschiebbar ist. In der Ausgangsstellung ist das Außenrohr 2 über ein Sperrelement 8 gegen das Verschieben in Axialrichtung gesperrt, das am proximalen Ende des Außenrohres 2 anliegt. Das Sperrelement 8 ist über einen an der Handhabe 4 angeordneten Betätigungsknopf 9 entriegelbar. Hierzu wird der Betätigungsknopf 9 gegen die Kraft einer Druckfeder 10 in das Gehäuse der Handhabe 4 eingedrückt. In einem Schaft 11 des Betätigungsknopfes 9 ist eine Kurvenbahn 12 ausgebildet, in der ein kraftschlüssig mit dem Sperrelement 8 verbundener Stift 13 gelagert ist. Wie aus Fig. 2 ersichtlich ist, ist die Kurvenbahn 12 im Schaft 11 des Betätigungsknopfes 9 so ausgebildet, daß beim Eindrücken des Betätigungsknopfes 9 das Sperrelement 8 über den in der Kurvenbahn 12 gelagerten Stift 13 gegen die Kraft einer Druckfeder 14 abwärts aus der das Außenrohr 2 verriegelnden Stellung gezogen wird. Am Ende der Kurvenbahn 12 ist ein Rastpunkt 15 ausgebildet, in dem der Betätigungsknopf 9 durch den am federbelasteten Sperrelement 8 ausgebildeten Stift 13 in der eingedrückten Stellung eingerastet verbleibt.

In dieser Stellung, in der das Sperrelement 8 das Außenrohr 2 freigegeben hat, steht das Außenrohr nur noch unter dem Federdruck einer Druckfeder 16, durch die das Außenrohr 2 in die Ausgangsstellung federbelastet ist.

Wird jetzt über eine in das Schneidrohr 3 eingeführte - nicht dargestellte - Faßzange ergriffenes Gewebe axial in Richtung zur Handhabe 4 gezogen, wird über das Gewebe das nunmehr relativ zum Schneidrohr 3 axial verschiebbare Außenrohr 2 gegen die Rückstellkraft der Feder 16 hin zur Handhabe 4 verschoben. Das Verschieben des Außenrohres 2 hin zur Handhabe 4 erfolgt soweit, bis in der in Fig. 1b dargestellten Arbeitsstellung die Schneide 5 des Schneidrohres 3 aus dem distalseitigen Ende des Außenrohres 2 hinausragt und über den Antrieb 6 angetrieben einen zylinderförmigen Gewebeblock aus dem über die Faßzange ergriffenen Gewebe herausschneidet.

Beim Zurückschieben des Außenrohres 2 wird das Sperrelement 8 über eine am proximalen Ende des Außenrohres 2 ausgebildete Anlaufschräge 17 weiter nach unten gedrückt, wodurch der am Sperrelement 8 ausgebildete Stift 13 aus dem Rastpunkt 15 der Kurvenbahn 12 austritt und der Betätigungsknopf 9 durch die Feder 10 wieder aus dem Gehäuse der Handhabe 4 herausgedrückt wird. Gleichzeitig drückt die das Sperrelement 8 in Sperrichtung belastende Feder 14 das Sperrelement 8 wieder aufwärts in Richtung der Sperrstellung.

Sobald der Druck auf das distalseitige Ende des Außenrohres 2 nachläßt, wird das Außenrohr 2 über die Feder 16 wieder in die die Schneide 5 des Schneidrohres 3 überdeckende Ausgangsstellung zurückgedrückt. Gleichzeitig mit dieser Axialverschiebung des Außenrohres 2 läuft das Sperrelement 8 gegen die Anlaufschräge 17 am proximalen Ende des Außenrohres 2 an und wird über die Feder 14 zurück in die in Fig. 2 dargestellte Sperrstellung am proximalen Ende des Außenrohres 2 überführt.

Für einen weiteren Schneidevorgang ist es notwendig, zuerst wieder den Betätigungsknopf 9 einzudrücken, um das Sperrelement 8 in die das Außenrohr 2 freigebende Entriegelungsstellung zu überführen.

Mit einem solchermaßen ausgebildeten Morcellator 1, bei dem das Außenrohr 2 und das Schneidrohr 3 relativ zueinander zwischen einer die Schneide 5 des Schneidrohres 3 vollständig durch das Außenrohr 2 abdeckenden Ausgangsstellung und einer Arbeitsstellung verschiebbar sind, in der die Schneide 5 des Schneidrohres 3 aus dem distalseitigen Ende des Außenrohres 2 hinausragt, wird sichergestellt, daß ein versehentliches Schneiden an ungewollten Stellen weitestmöglich verhindert wird, da ein Schneiden nur möglich ist, wenn über die Betätigung des Betätigungsknopfes 9 die Verriegelung der Ausgangsstellung freigegeben wird.

Neben der dargestellten und beschriebenen Möglichkeit, das Außenrohr 2 verschiebbar zu lagern und das Schneidrohr 3 festzuhalten, ist es selbstverständlich auch möglich, bei einem in axialer Richtung starren Außenrohr 2 das Schneidrohr 3 zwischen der Ausgangsstellung und der Arbeitsstellung verschiebbar im Außenrohr 2 zu lagern.

Während der voranstehend beschriebene Morcellator 1 dazu bestimmt ist, direkt, d.h. ohne Trokar in den Körper bzw. die Körperhöhle eingeführt zu werden, ist die beschriebene Anordnung von Außenrohr 2 und Schneidrohr 3, die relativ zueinander verschiebbar sind, auch für Morcellatoren 1 anwendbar, die durch einen zusätzlichen Trokar einzuführen sind.

Das Operieren mit dem Morcellator 1 gemäß den Abbildungen Fig. 1a, 1b und 2 erfolgt folgendermaßen:

Zur Operation wird der Morcellator 1 auf den Körper aufgesetzt und durch den freien Querschnitt des Schneidrohres 3 ein Trokardorn eingeführt. Mit diesem Trokardorn wird dann die Haut durchstochen, um zu dem bereits vorbereiteten Operationsgebiet in der Körperhöhle zu gelangen. Durch den so geschaffenen Einstichkanal wird dann das Außenrohr 2 des Morcellators 1 in die Körperhöhle eingeführt und der Trokardorn wieder aus dem Morcellator 1 herausgezogen.

Unter Sichtkontrolle über ein endoskopisches Sichtinstrument, welches über einen weiteren Einstichkanal in die Körperhöhle eingeführt ist, wird der Morcellator 1 hin zum Operationsgebiet bewegt. Zur eigentlichen Operation wird nun durch den freien Querschnitt des Schneidrohres 3 die Faßzange eingeführt. Das zu entfernende Gewebe wird mit der Faßzange ergriffen und die Faßzange wieder zurück in das Schneidrohr 3 gezogen.

Um das Ausschneiden eines zylinderförmigen Gewebeblocks mittels der Schneide 5 des Schneidrohres 3 zu ermöglichen, wird das Außenrohr 2 durch Drücken des Betätigungsknopfes 9 entriegelt. Durch das Ziehen des mit der Faßzange ergriffenen Gewebes gegen das distale Ende des Außenrohres 2 wird dieses jetzt in axialer Richtung hin zur Handhabe 4 verschoben, bis in der Arbeitsstellung die Schneide 5 über das distalseitige Ende des Außenrohres 2 hinausragt. Über die Schneide 5 des angetriebenen Schneidrohres 3 wird jetzt ein zylinderförmiger Gewebeblock aus dem mit der Faßzange ergriffenen Gewebe herausgeschnitten.

Sobald nach dem Ausschneiden des Gewebeblocks der Druck auf das distale Ende des Außenrohres 2 nachläßt, drückt die Feder 16 das Außenrohr 2 wieder zurück in die Ausgangsstellung über das Schneidrohr 3 zurück.

Ein weiteres Schneiden mittels des Schneidrohres 3 ist nur möglich, wenn das Außenrohr 2 wieder entriegelt und durch das von der Faßzange ergriffene Gewebe in die Arbeitsstellung verschoben wird.

### Bezugszeichenliste

- 1: Morcellator
- 2: Außenrohr
- 3: Schneidrohr
- 4: Handhabe
- 5: Schneide
- 6: Antrieb
- 7: Längsachse
- 8: Sperrelement
- 9: Betätigungsknopf
- 10: Feder
- 11: Schaft
- 12: Kurvenbahn
- 13: Stift
- 14: Feder
- 15: Rastpunkt
- 16: Feder
- 17: Anlaufschräge

## Patentansprüche

1. Instrument zum Schneiden von biologischem und insbesondere menschlichem Gewebe, insbesondere bei einem endochirurgischen Eingriff, mit einem Außenrohr (2) und einem in dem Außenrohr (2) angeordneten, um seine Längsachse (7) drehbaren hohlzylindrischen Schneidrohr (3), an dessen distalem Ende mindestens eine Schneide (5) angeordnet ist, sowie mit einer am proximalen Ende der Rohre (2, 3) angeordneten Handhabe (4),wobei das Außenrohr (2) in Axialrichtung des unverschiebbar an der Handhabe (4) festgelegten Schneidrohres (3) gegen die Kraft einer Druckfeder (16) zwischen einer Ausgangsstellung, in der das Außenrohr (2) das Schneidrohr (3) distalseitig überragt, und einer Arbeitsstellung, in der die Schneide (5) aus dem distalseitigen Ende des Außenrohres (2) hinausragt, verschiebbar ist,
**dadurch gekennzeichnet,**
**daß** das Außenrohr (2) in der Ausgangsstellung über ein Sperrelement (8) gegen die Verschiebung in Axialrichtung gesperrt ist, das am proximalen Ende des Außenrohres (2) anliegt und über einen an der Handhabe (4) angeordneten Betätigungsknopf (9) derart entriegelbar ist, daß das Außenrohr (2) anschließend unabhängig von einer weiteren Betätigung des Betätigungsknopfes (9) durch Ausüben einer axialen Druckkraft auf das Außenrohr (2) in die Arbeitsstellung verschiebbar ist, und daß die Druckfeder (16) das Außenrohr (2) nach jeder Überführung in die Arbeitsstellung automatisch wieder in die gesperrte Ausgangsstellung überführt.

2. Instrument nach Anspruch 1, **dadurch gekennzeichnet, daß** das Sperrelement (8) in Richtung der Sperrstellung federbelastet ist.

3. Instrument nach Anspruch 1 oder 2, **dadurch gekennzeichnet, daß** am proximalen Ende des Außenrohres (2) eine Anlaufschräge (17) für das Sperrelement (8) ausgebildet ist.

4. Instrument nach einem der Ansprüche 1 bis 3, **dadurch gekennzeichnet, daß** das Entriegeln des Sperrelementes (8) über einen fest mit dem Sperrelement (8) verbundenen Stift (13) erfolgt, der in einer in einem Schaft (11) des Betätigungsknopfes (9) ausgebildeten Kurvenbahn (12) gelagert und beim Drükken des Betätigungsknopfes (9) entlang dieser Kurvenbahn (12) verfahrbar ist.

5. Instrument nach einem der Ansprüche 1 bis 4, **dadurch gekennzeichnet, daß** der Betätigungsknopf (9) gegen die Kraft einer Druckfeder (10) in die Entriegelungsstellung ins Gehäuse der Handhabe (4) eindrückbar ist.

## Claims

1. Instrument for cutting biological and in particular human tissue, in particular in an endoscopic surgical procedure, with an outer tube (2), and with a hollow cylindrical cutting tube (3) which is arranged in the outer tube (2) and can rotate about its longitudinal axis (7) and at whose distal end at least one cutter (5) is arranged, and with a handle (4) arranged at the proximal end of the tubes (2, 3), the outer tube (2) being displaceable in the axial direction of the cutting tube (3), which is fixed non-displaceably on the handle (4), counter to the force of a compression spring (16) between a starting position, in which the outer tube (2) projects beyond the cutting tube (3) at the distal end, and a working position, in which the cutter (5) protrudes from the distal end of the outer tube (2), **characterized in that**, in the starting position, the outer tube (2) is locked against the displacement in the axial direction by a locking element (8) which bears on the proximal end of the outer tube (2) and can be unlocked via an actuating button (9), arranged on the handle (4), in such a way that the outer tube (2) can then be displaced into the working position, independently of further actuation of the actuating button (9), by exerting an axial pressure force on the outer tube (2), and that, after each transfer to the working position, the compression spring (16) automatically returns the outer tube (2) to the locked starting position.

2. Instrument according to Claim 1, **characterized in that** the locking element (8) is spring-loaded in the direction of the locking position.

3. Instrument according to Claim 1 or 2, **characterized in that** a contact bevel (17) for the locking element (8) is formed at the proximal end of the outer tube (2).

4. Instrument according to one of Claims 1 to 3, **characterized in that** the locking element (8) is unlocked via a pin (13) which is securely fixed to the locking element (8) and which is mounted in a curved track (12) formed in a shaft (11) of the actuating button (9) and can be moved along this curved track (12) upon pressing the actuating button (9).

5. Instrument according to one of Claims 1 to 4, **characterized in that** the actuating button (9) can be pressed into the housing of the handle (4), counter to the force of a compression spring (10), into the unlocking position.

## Revendications

1. Instrument pour couper des tissus biologiques et en particulier des tissus humains, notamment lors d'une intervention endo-chirurgicale, comportant un tube extérieur (2) et un tube de coupe cylindrique creux (3) qui est agencé dans le tube extérieur (2) et susceptible de tourner autour de son axe longitudinal (7) et dont l'extrémité distale comprend au moins un tranchant (5), et comportant une manette (4) agencée à l'extrémité proximale des tubes (2, 3), le tube extérieur (2) étant mobile en direction axiale du tube de coupe (3), fixé de façon immobile sur la manette (4), à l'encontre de la force d'un ressort de compression (16) entre une position initiale dans laquelle le tube extérieur (2) dépasse du côté distal au-delà du tube de coupe (3) et une position de travail dans laquelle le tranchant (5) dépasse hors de l'extrémité côté distal du tube extérieur (2), **caractérisé en ce que** le tube extérieur (2) est bloqué dans la position initiale à l'encontre du déplacement en direction axiale via un élément de blocage (8) qui s'appuie à l'extrémité proximale du tube extérieur (2) et qui peut être déverrouillé via un bouton d'actionnement (9) agencé sur la manette (4), de telle sorte que le tube extérieur (2) est alors mobile jusque dans la position de travail indépendamment d'une poursuite de l'actionnement du bouton d'actionnement (9) par exercice d'une force de pression axiale sur le tube extérieur (2), et **en ce que** le ressort de compression (16) ramène automatiquement le tube extérieur (2), après chaque transfert dans la position de travail, de nouveau jusque dans la position initiale bloquée.

2. Instrument selon la revendication 1, **caractérisé en ce que** l'élément de blocage (8) est chargé par un ressort en direction de la position bloquée.

3. Instrument selon l'une ou l'autre des revendications 1 et 2, **caractérisé en ce qu'**une pente de montée (17) pour l'élément de blocage (8) est réalisée à l'extrémité proximale du tube extérieur (2).

4. Instrument selon l'une des revendications 1 à 3, **caractérisé en ce que** le déverrouillage de l'élément de blocage (8) s'effectue via une goupille (13) fermement reliée à l'élément de blocage (8), laquelle est montée dans une trajectoire incurvée (12) réalisée dans une tige (11) du bouton d'actionnement (9) et qui est mobile le long de cette trajectoire (12) lors de l'enfoncement du bouton d'actionnement (9).

5. Instrument selon l'une des revendications 1 à 4, **caractérisé en ce que** le bouton d'actionnement (9) peut être enfoncé à l'encontre de la force d'un ressort de compression (10) dans le boîtier de la manette (4) jusque dans la position de déverrouillage.
